# EUROPEAN PATENT APPLICATION

(11) **EP 1 057 498 A2**
(43) Date of publication of application: **06.12.2000**
(21) Application number: 00850094.4
(22) Date of filing: 26.05.2000
(51) Int. Cl.: A61N 1/39, A61B 5/024, A61B 5/05

(54) **System and method for measuring blood flow during defibrillation**

(30) Priority: 31.05.1999 NO 992613
(71) Applicant: Laerdal Medical AS, 4001 Stavanger (NO)
(72) Inventor: Myklebust, Helge, 4025 Stavanger (NO)
(74) Representative: Hammar, Ernst

(57) **Abstract**

Method, device for use during resuscitation following sudden cardiac death, and also a device for measuring the blood stream in tissue, including electrode supports with a first set of electrodes designed to be placed on the outside if the skin, is supplied with an approximately constant current from an AC source, and the voltage between said set of electrodes, or possibly from a second set of electrodes that is placed on the skin in close proximity to the first set, and which is supported by separate electrode supports or electrode supports that are shared with the first set, is measured by a voltage metering device (7) to which is connected signal processing means for deduction of respiratory signals and pulse signals or blood stream signals and signals to indicate chest compression. The constant AC source can supply a signal at a frequency from about 1 kHz to about 10 MHz, preferably at a frequency near 30 kHz.

The device for measuring the blood stream may be connected in series with a compression pad containing an accelerometer, as a display screen may also be connected up in series with said device in order to show compression rate and depth of compressions, pulse and ECG, and the device may have a connector connected to a modem, for connecting up to e.g. an emergency centre, a computer for further processing of the data, and/or an AED. The device for measuring the blood stream may also be integrated into a defibrillator.

## Description

The present invention relates to a method for an external defibrillator that measures a blood stream by impedance analysis as stated in the introduction to Claim 1, and also a system for implementation of the method as stated in the introduction to Claim 7.

In the case of sudden cardiac death, it is essential that life-saving measures be started immediately, and that the correct treatment is initiated. One of the most important parameters is the pulse, which is to be checked according to standard resuscitation guidelines, as the question of whether a pulse is present or not decides whether cardiopulmonary resuscitation and defibrillation should be carried out.

The most common technique for finding the pulse is to feel the outside of the skin and apply a slight pressure using two fingers just over a carotid artery. Medical literature demonstrates the fact that this measurement method is both uncertain and time-consuming. Even operators with medical experience may find it problematic to perform a pulse check quickly and reliably.

Examples exist of equipment that may be used to check for a pulse. The most commonly used is the pulse-oxymeter, which measures the oxygen saturation of the blood through illuminating the skin, and which is normally capable of indicating the presence of a blood stream. However this equipment does not give reliable results when the patient has a weak pulse or when the blood vessels in the skin have contracted due to the patient being cold or having been given medication such as adrenaline. Other types of equipment employ the ultrasound-Doppler principle or the ultrasound-sonar principle. The correct positioning and angle relative to the artery determine the reliability of the measurements when using this type of equipment. It may be difficult for the instruments to distinguish between blood flowing through the artery and blood that is pulsating back and forth. It is also a disadvantage that this equipment constitutes an addition to equipment that is already in use, as it will require extra time or alternatively extra personnel to operate.

Making use of impedance variations in order to measure a blood stream, and thereby the pulse, is based on known principles, described as early as the 1920s and 30s. By running an approximately constant alternating current through two electrodes fixed to the body, a voltage can be measured between two points located between the electrodes. according to the formula **U = Z x I**, in which **U** is the voltage, **Z** is the impedance and **I** is the current. Changes in the impedance will then emerge as changes in the voltage as measured between the electrodes.

It has been proven that the resistivity of blood, measured along the direction of flow, is reduced as the velocity of the blood increases. The voltage change is also caused by the arteries expanding with each beat of the pulse, so as to give a relative increase in the volume of blood in the tissue.

If the electrodes are fixed to the chest, breathing will also modulate the voltage measured. This is caused by the impedance (thorax impedance) between the electrodes increasing upon inhalation of air, by the current path lengthening as the lungs are filled with air.

The most common areas of use for impedance variations in tissue are:
1. Measuring breathing, estimation of inhaled volume as a function of the change in thorax impedance.
2. Measuring the blood stream in the extremities as a function of the change in tissue impedance is known as "Impedance Plethysmography" (IP).
3. Measuring the cubic capacity of the heart as a function of the change in thorax impedance in phase with the ECG is known as "Impedance Cardiography" (IC).

In addition, experiments on animals have shown that chest compression is associated with an increase in impedance.

The above principles may be used in combination in situations of sudden cardiac death, in order to perform pulse checks, estimate the volume of blood delivered by each heart heat and cerebral blood stream (blood stream to the brain), as well as to demonstrate and document the use of heart compression during the treatment.

Despite the above having been known for a long time, no attempts have been made to make use of these principles in connection with resuscitation.

The object of the present invention is to avoid the disadvantages of alternative techniques and principles of blood stream measurements and pulse checks as mentioned at the beginning. This is achieved by using the change in tissue impedance to determine the blood stream, more particularly through a method of the type mentioned at the beginning, the characteristics of which can be seen from Claim 1, and also a system for implementation of the method, of the type mentioned at the beginning, the characteristics of which can be seen from Claim 7. Further characteristics of the invention can be seen from the remaining, dependent claims.

Thus, the present invention as stated in the claims allows impedance measurements to be used in connection with sudden cardiac death and resuscitation, where the blood stream signal is used to replace the pulse check. This saves time; time meaning a greater chance of saving life.

Furthermore, the above-mentioned will make the equipment portable and miniaturised in comparison with laboratory equipment that is commercially available.

Measuring of blood stream signals facilitates documentation of the result of the resuscitation attempts.

The device of the invention, which device uses measurements of change in impedance, may be a stand-alone unit, a unit co-operating with a defibrillator (AED), in which case it is connected in series with the cable to the AED, or a unit integrated into the AED.

An impedance measurement combined with an AED has the advantage of allowing the confirmation of pulse/no pulse to be carried out in parallel with the ECG analysis, which means that the pulse measurement can be conducted without reducing the time available for treating the patient. This is of great advantage to the patient, as further treatment can be initiated more quickly. As an example; if the patient has an ECG of the type Pulseless Electrical Activity (PEA), a condition in which there is no measurable pulse and a defibrillator shock would have no effect, the AED takes approximately 10 seconds to inform the lifesaver that CPR must be initiated. According to medical literature, this is only a tenth of the time it takes for operatives to initiate CPR under identical conditions but with manual pulse check.

Furthermore, it will be possible to use common electrodes and electronics when the device is integrated in a defibrillator. This is highly advantageous to the user, as there is only one apparatus that is to be connected up and used during the life-saving operation.

When compared with manual pulse checks and blood stream measurements based on ultrasound, the positioning is not critical for impedance measurements. It is not required that the electrodes be glued directly over an artery. In case of sudden cardiac death and resuscitation, the body will prioritise blood to the head, chest and lungs, while the extremities receive less blood. Thus the device is suitable for measuring the blood stream both in the chest and to the head.

Using impedance measurements in combination with an accelerometer may improve the accuracy of the measurements considerably, i.e. erroneous readings are avoided through the patient's movements being indicated, as movements interfere with impedance measurements. When measuring the acceleration, this input signal may in addition be used as a noise signal to an adaptive filter, which will improve the signal-to-noise ratio, and thus the accuracy of the measurement. The accelerometer measuring unit may be of the type described in Norwegian Patent Application no. 1996 2611, filed at the same time as the present application, with the same applicant.

Impedance measuring will be able to replace manual pulse checks, by the amplitude of the blood stream signal determining whether the pulse should be described as strong. weak or no pulse, besides which it will be possible to count the pulse rate.

In the following, the invention will be described in greater detail with reference to the drawings, in which:
- Fig. 1: schematically shows the principle of an impedance measuring system integrated into a defibrillator;
- Fig. 2a: shows a resuscitation flow diagram, where the defibrillator makes use of information from the invention in order to select treatment, or alternatively the user makes use of information from the invention in order to make decisions regarding further treatment;
- Fig. 2b: shows a flow diagram in accordance with existing resuscitation guidelines, in which the user must select the type of treatment based upon a manual pulse check;
- Figs. 3a-3b: show two examples of embodiments when the invention is provided as supplementary equipment to a defibrillator
- Figs. 4a-4b: show two examples of embodiments when the invention is arranged integrated into a defibrillator; and
- Figs. 5a-5d: show curves representing measurements carried out on animals.

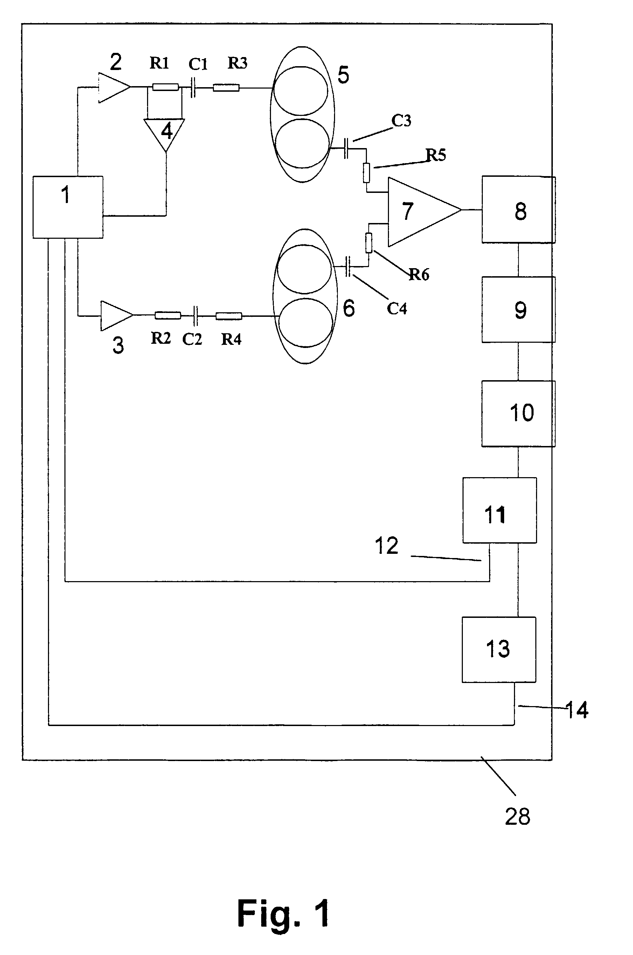

In the following, a circuit for possible implementation of the invention will be described in greater detail with reference to Figure 1.

Figure 1 illustrates the principle of the impedance measuring system 28 by a CPU unit 1 transmitting an alternating voltage to driver amplifiers 2, 3, where the output signals from the drivers 2, 3 are 180° out of phase relative to each other. The alternating voltage preferably has a frequency of 30 kHz, however the frequency may range from about 1 kHz to more than 10 MHz.

The amplifiers 2, 3 drive the signal with a low output impedance, and have sufficient current capacity.

Reference no. 4 indicates an amplifier, which together with CPU 1 and driver amplifiers 2, 3 sets the current to be nearly constant. Constant current is provided by a resistor R1, which is connected in series with the one electrode 5, being connected to the output from the amplifier 2. The current through the body between the electrodes 5 and 6 is measured by the voltage before and after said resistor being introduced to the respective inlets to a differential amplifier 4, and then to the CPU 1 for analysis. If the measured current falls, the CPU 1 will increase the voltage to the driver amplifiers 2, 3, until the output from the differential amplifier 4 again indicates the selected current value. Likewise, if the measured current increases, CPU 1 will reduce the voltage to the driver amplifiers 2, 3 until the output from the differential amplifier 4 again indicates the selected current value. Intermittent contact between the electrodes 5, 6 and the skin is the main reason for fluctuating current. The current limits are 3 mA for 30 kHz, 4 mA for 40 kHz etc. but no higher than 10 mA in accordance with the specifications given in IEC standard 601-1, "Medical electrical equipment, Part 1: General requirements for safety". The components R1, R2, R3, R4 together with C1 and C2 set the impedance seen from the electrodes to a sufficiently high values, such that the connection of the amplifiers 2, 3 to the circuit only constitutes an insignificant short-circuiting of the ECG signal, which is also measured by the defibrillator through an ECG measuring system 23 connected to the electrodes 5, 6. At the same time, the components R1, R2, R3, R4, C1, C2 protect the amplifiers 2, 3 against the high voltage that is delivered to the electrodes by the shock delivery circuit 22 when the patient is defibrillated.

The electrodes 5,6 are designed with an electrically conductive surface that connects the high voltage, the AC source and the measuring systems to the patient's skin, alternatively each of the electrodes 5,6 consists of two or more electrically conductive surfaces, where one surface is preferably used by systems that measure signals and one surface is used by systems that deliver electrical energy to the patient, either in the form of alternating current or high voltage. The advantage of letting the measuring systems use separate conductive surfaces towards the patient is the fact that this to a larger extent avoids signal interference caused by intermittent electrical contact across the electrode part that connects the patient to the AC source. The patient moving primarily causes such intermittent electrical contact.

A differential amplifier 7 is arranged after the electrodes 5, 6, which amplifier measures the voltage difference between the electrodes. RC elements consisting of C3 and R5, and C4 and R6 respectively, are arranged at the inlets to the differential amplifier 7, which elements form high-pass filters for filtering out DC components, as well as for reducing interference from the ECG signals. Another high-pass filter 8 is connected to the differential amplifier 7 for further suppression of the ECG signal components. The signal from said high-pass filter 8 is amplified in an AC-connected amplifier 9. The outlet of the AC amplifier 9 is connected to a precision rectifier block 10 that demodulates the AC signal. From rectifier 10, the signal is transmitted to a circuit 11 that removes the DC component and amplifies the signal, which in this case is proportional to a patient's breathing when the electrodes are fixed to the chest. This signal 12 is passed on to an indicator unit for direct display of the signal or to CPU 1 for further processing in order to give a readable indication, or to storage for subsequent retrieval in order to provide a statement regarding the course of events during the lifesaving operation.

The signal from the above circuit 11 is also sent on to another circuit 13, which again removes the DC component and the low frequency components and amplifies the signal that represents the change in impedance due to the blood stream. This signal 14 is then sent on either to an indicator unit or to CPU1 for further processing in order to give a readable indication, or to storage for subsequent retrieval in order to provide a statement regarding the course of events during the lifesaving operation.

A more detailed description of the principle and how the various signals can be measured:

Between the electrodes of the defibrillator, the impedance that can be measured with an alternating current of around 30 kHz will lie somewhere between 40 ohms and 150 ohms, with a default value of around 80 ohms. Breathing or artificial ventilation will cause small variations in this impedance. A normal inhalation will cause a change in impedance in the order of 1 ohm. In addition, each heartbeat will cause a change in impedance in the order of 10 milliohms.

If the system uses an approximately constant alternating current for which the mean value of each alternation is 1 mA, the signal between the electrodes will be measurable as:

| | | |
|---|---|---|
| | 1 mA ·80 ohms | static impedance |
| + | 1 mA ·(+,-) 0.5 ohms | changes due to breathing |
| + | 1 mA ·(+/-) 5 mohms | changes due to the blood stream |
| + | EKG | where the amplitude is normally around 1 mV. |

As can be seen from the listing, the signal component due to breathing will be of approximately the same magnitude as the signal component due to the ECG. Furthermore, the signal component due to the blood stream will only constitute one hundredth of the signal component due to breathing.

The strategy for signal processing is thus: Because there is a relatively large difference in frequency between the ECG (here, the energy lies in the area 2 - 40 Hz) and the signal created by the approximately constant alternating current (preferably 30 kHz), it will be possible, through band-pass filtering of the impedance signal, to suppress the ECG contribution sufficiently. This band-pass filtering takes place through use of the RC elements C3-R5 and C4-R6, together with filter step 8. Once the ECG contribution has been dampened sufficiently, an AC connected amplifier 9 will, together with the demodulator 10 in the form of a precision rectifier block, be able to create a basis for further signal analysis. At the outlet from 10, the signal will consist of a DC component that is proportional to the static impedance of the patient (normally 80 ohms), where a ripple has been overlaid, which ripple is proportional to any breathing, and where a further ripple component has been overlaid, which ripple component is proportional to any blood stream. As these individual components are different with regards to frequency, it is possible through use of classic signal processing to amplify and filter the signal in such a way as to separate the individual signal components.

In the case of sudden cardiac death, there will be no signal components from breathing or the blood stream. Only when artificial respiration is administered to the patient, will this appear as a signal on the respiration channel 12. The primary objective of the invention is to quickly be able to determine whether a given defibrillator shock has had the desired effect. If the heart starts to beat again, this will primarily become visible through blood stream channel 14 showing a signal in phase with the electrical activity of the heart, the ECG. The patient will be able to breathe unaided only after some time has passed.

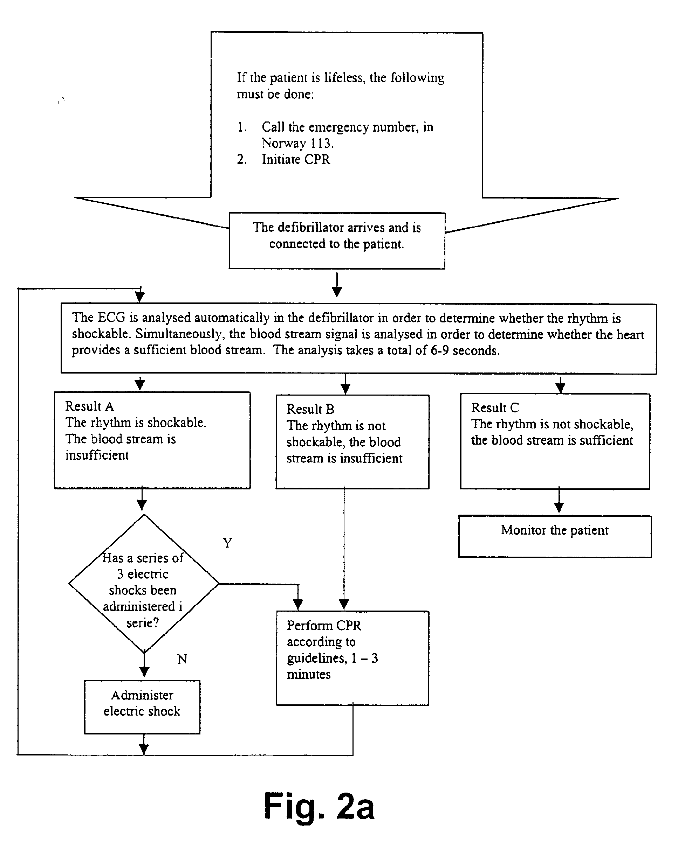

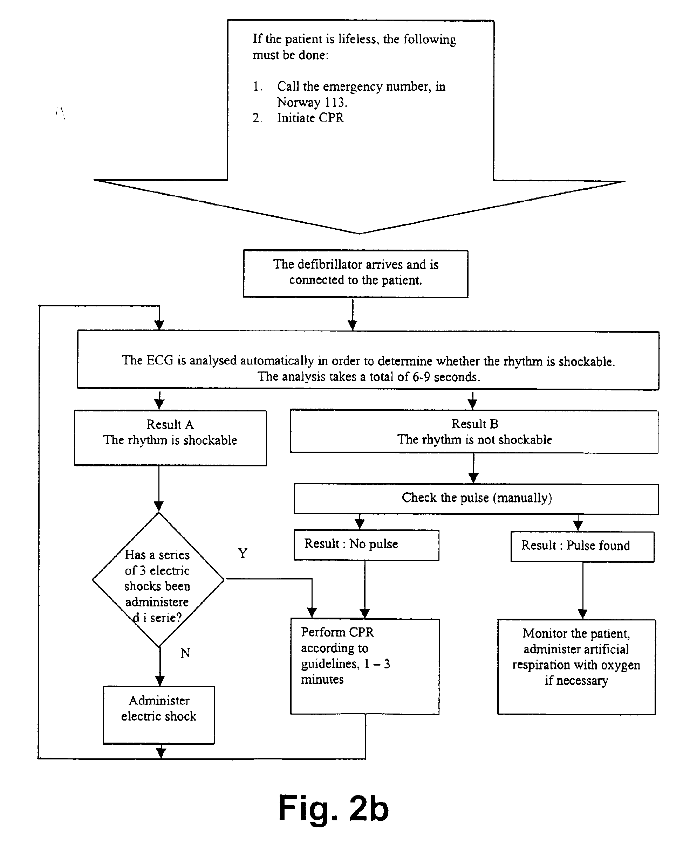

Figures 2a-2b show flow diagrams (treatment protocols) for resuscitation, with and without the use of an automated pulse check based on blood stream measurements. In figure 2a, the blood stream measurement will be carried out in parallel with the automatic ECG analysis, something that the defibrillator still takes 6-9 seconds to perform. In this case, the pulse measurement will be automated, objective and accurate. In Figure 2b, which is in accordance with the existing resuscitation guidelines, the pulse is to be checked manually every time the ECG analysis concludes that the rhythm is so-called non-shockable. "Advisory Statements of the International Liaison Committee on Resuscitation (ILCOR)" maintains that a manual pulse check must be performed over a period of more than 30 seconds in order for the diagnosis to have a diagnostic accuracy of at least 95%. Another publication (Eberle B, et al; Resuscitation 1996; 33: 107-16) demonstrates that only 15% of the participants were able to conclude the correct treatment based on a manual pulse check performed over 10 seconds. For the patient, this means that the choice of treatment will either be based on chance, without diagnostic precision, or the patient will have to remain untreated for more than 30 seconds while the diagnosis is being made.

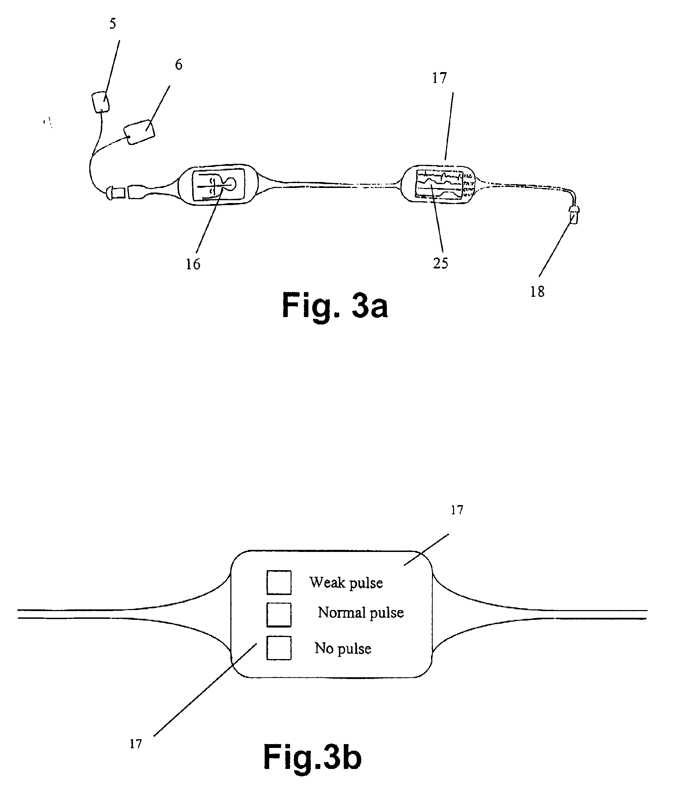

Figure 3a shows an embodiment in which a compression pad 16 with an accelerometer is connected in series with a pulse unit 17 with a display screen 25. The following may be displayed on said screen: Compression rate and depth of compressions, a curve indicating the blood stream signal 14, a curve indicating the respiratory signal 12, as well as ECG. The unit is connected to the defibrillator via the connector 18. Figure 3b shows an alternative embodiment, in which the display screen is replaced by indicators 26 that describe the pulse as either absent, weak or good, and which will help the user choose the correct form of treatment, see also Figure 2b.

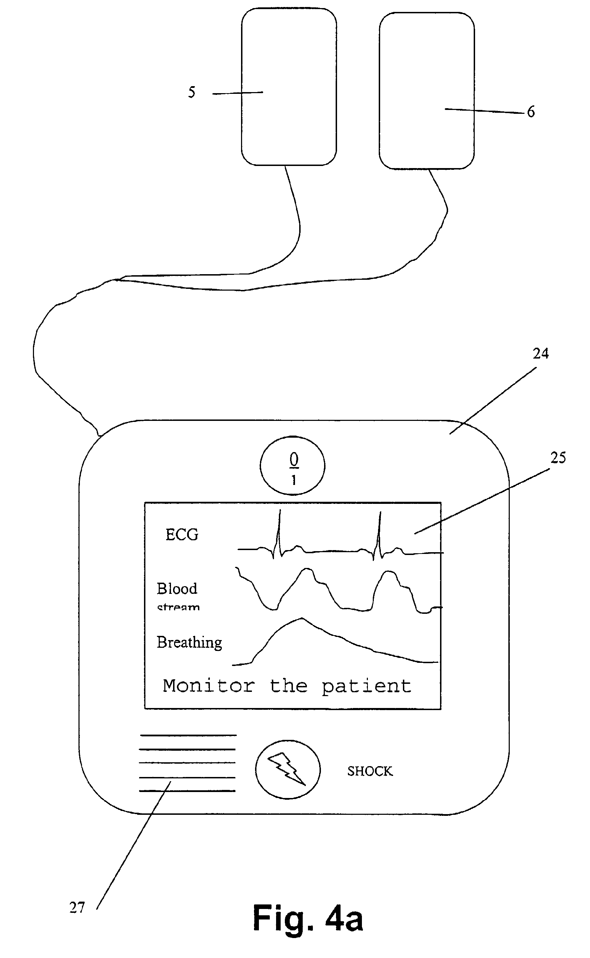

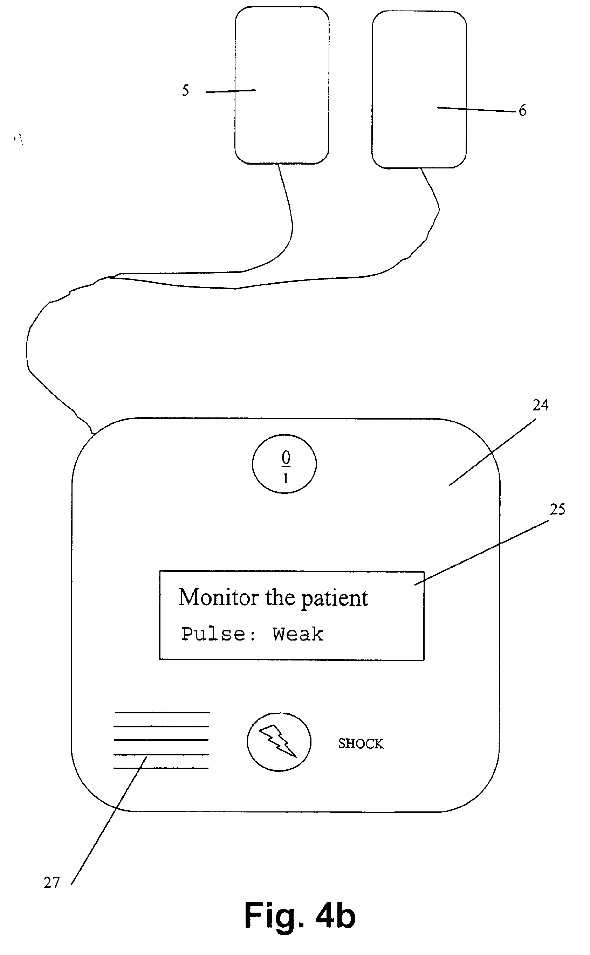

Figure 4a shows an example of an embodiment where the invention is integrated in a defibrillator. The defibrillator display screen 25 shows the information from the impedance measuring system in the form of curves for the blood stream signal and the respiratory signal respectively. In addition, a text message is displayed to instruct the user regarding the action to be taken according to the resuscitation flow diagram (see Figure 2a). Figure 4b shows an alternative embodiment, in which the curves have been replaced by text messages; a solution aimed at users who to a greater extent leave the defibrillator to interpret the condition of the patient. Today, all defibrillators are equipped with a sound unit 27, in addition to the display unit, which sound unit generates both sound signals and voice messages for the user. The defibrillator makes use of the sound unit in order to guide the lifesaver in accordance with the treatment protocol, see flow diagram Figure 2a.

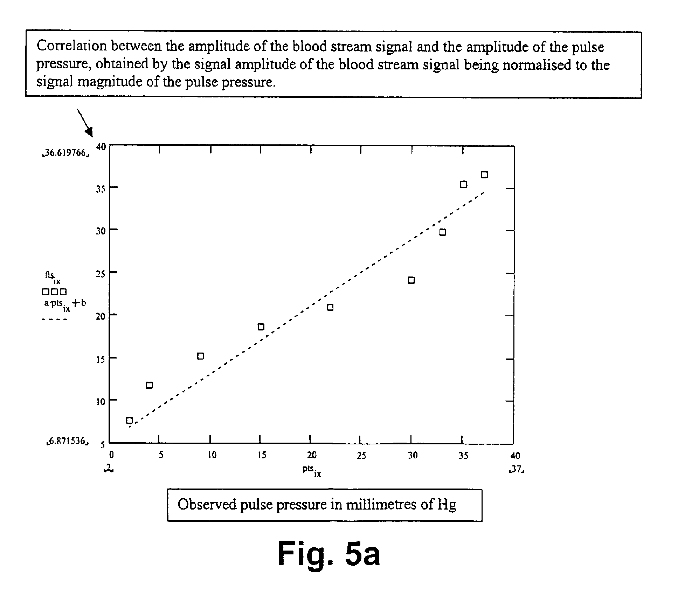

Figure 5a shows a curve indicating the correlation between the amplitude of the pulse pressure and the amplitude of the blood stream signal for one animal. The pulse pressure is defined as the difference between the systolic pressure and the diastolic pressure in an artery.

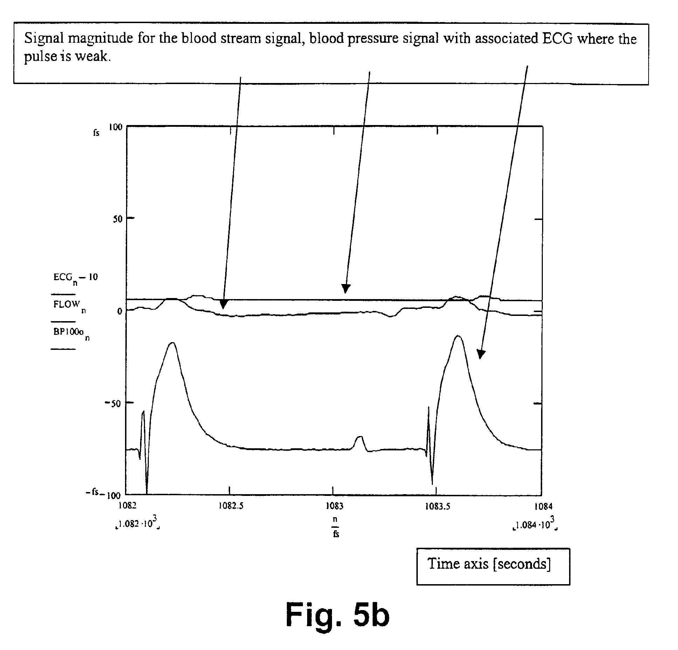

Figure 5b shows the ECG (bottom), the blood stream signal (middle) and the blood pressure (top) for an animal with a low blood pressure.

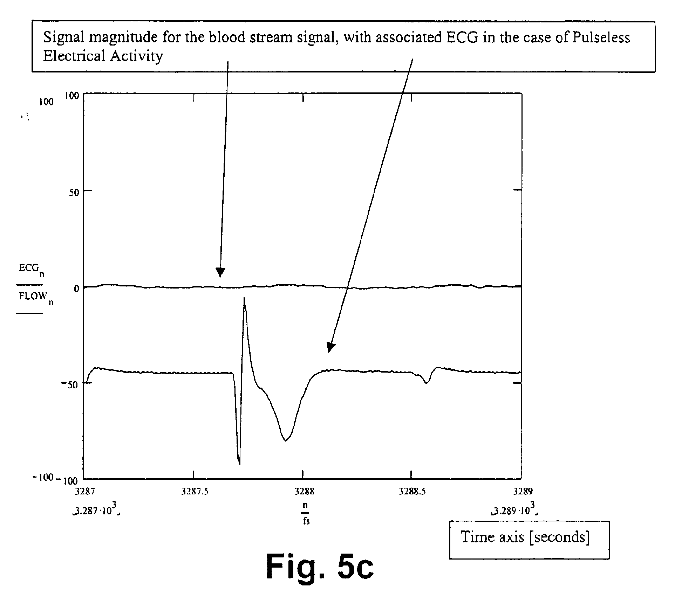

The curves in Figure 5c show the ECG (bottom) and the blood stream signal (top), where the ECG is of the type PEA (Pulseless Electrical Activity). The blood stream signal indicates no blood stream.

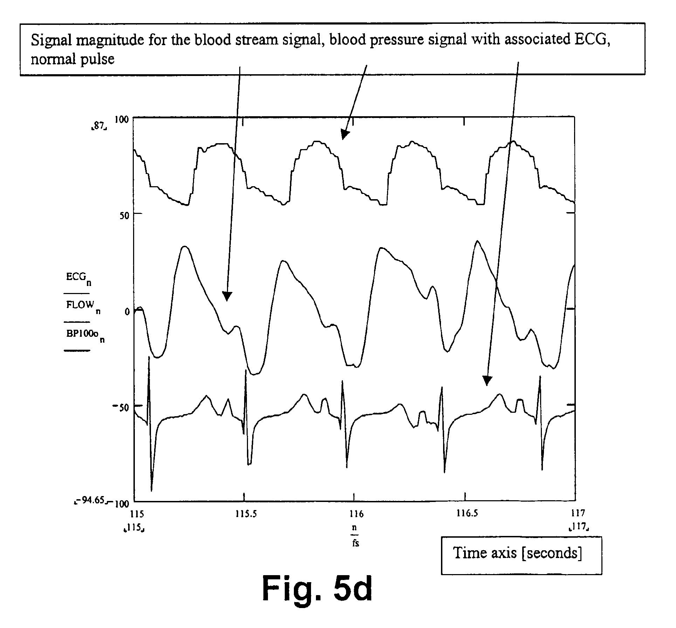

The curves in Figure 5d show a normal ECG (bottom), a normal blood stream signal (middle) and a normal blood pressure signal (top).

## Claims

1. Method of determining whether a lifeless person has a pulse, based on impedance measurement between electrodes placed on the patient's skin, in which the electrodes are connected to the impedance measuring system of an external defibrillator for a resuscitation attempt, **wherein** changes in impedance caused by changes in the patient's blood stream are amplified and registered by a method that is known *per se*, through an amplifier and a filter device integrated into the impedance measuring system of a defibrillator, in which the impedance measuring system has a signal output that represents the changes in the patient's blood stream as a function of time, the patient's pulse is classified in a calculation unit connected to the signal output of the impedance measuring system, on the basis of specific values for impedance change, and the choice of defibrillator programme is decided in the defibrillator programme unit by using the classification result, which programme unit is connected to the output of the calculation unit.

2. Method according to Claim 1, **wherein** the classification is defined as normal, weak or absent pulse.

3. Method according to Claims 1, **wherein** the filter device in the impedance measuring system is designed to dampen the contributions to impedance change caused by breathing and/or artificial respiration, while the impedance measuring system is designed to amplify the contributions from the blood stream changes, and where the impedance measuring system has a resolution of no less than 1 milliohm

4. Method according to Claims 1, **wherein** the pulse classification is performed simultaneously with the classification of the patient's ECG.

5. Method according to Claim 4, **wherein** a signal representing changes in the blood stream and/or the classification result is sent to a display or indicator unit in the form of light-emitting diodes and/or a display screen.

6. Method according to Claims 1, **wherein** a signal representing changes in the blood stream and/or the classification result is stored externally.

7. System for implementing the method of determining whether a lifeless person has a pulse, based on impedance measurements between electrodes placed on the patient's skin during a resuscitation attempt, **wherein** a defibrillator's impedance measuring system includes an integrated amplifier and a integrated filtering device that are know *per se,* for amplification and registration of changes in the patient's blood stream, where the impedance measuring system has a signal output that represents changes in the patient's blood stream as a function of time, which output is connected to a calculation unit that classifies the pulse based on specific values of impedance change, and where the classification result decides the choice of defibrillator programme by the defibrillator programme unit being connected to the output from the calculation unit.

8. System according to Claim 7, **wherein** the amplifier and the filtering device are arranged externally in relation to the defibrillator.

9. System according to Claims 7, **wherein** the calculation unit is connected to an accelerometer that is attached to the patient, where the calculation unit is designed to perform digital, adaptive filtering in which the signal from the accelerometer is used as a reference signal, or the calculation unit is designed to decide the validity of the impedance measurement based on the amplitude of the signal from the accelerometer.

10. System according to Claims 7, **wherein** is provided an indicator unit in the form of light-emitting diodes and/or a display screen, on which the result of the classification is indicated.
